# EUROPEAN PATENT APPLICATION

(11) **EP 3 848 342 A1**
(43) Date of publication of application: **14.07.2021**
(21) Application number: 19856541.8
(22) Date of filing: 02.09.2019
(51) Int. Cl.: C07B 33/00, B01J 23/34, C01G 45/02, C07B 41/06, C07B 41/08, C07B 45/04, C07C 45/38, C07C 45/39, C07C 47/54, C07C 49/78, C07C 49/786, C07C 303/36, C07C 311/16, C07C 311/29, C07D 307/46, C07B 61/00

(54) **METHOD FOR PRODUCING OXIDE USING ?-MANGANESE DIOXIDE**

(30) Priority: 07.09.2018 JP 2018167523
(71) Applicant: Tokyo Institute of Technology, Tokyo 152-8550 (JP)
(72) Inventor: HARA, Michikazu, Tokyo 152-8550 (JP); KAMATA, Keigo, Tokyo 152-8550 (JP); HAYASHI, Eri, Tokyo 152-8550 (JP); YAMAGUCHI, Yui, Tokyo 152-8550 (JP)
(74) Representative: J A Kemp LLP
(86) International application number: PCT/JP2019/034432
(87) International publication number: WO 2020/050215

(57) **Abstract**

With the object of efficiently producing an oxidation product, the present invention provides a method for producing an oxidation product by oxidizing a raw material compound in the presence of oxygen, wherein the raw material compound is oxidized in the presence of manganese dioxide having a crystal structure of β-type.

## Description

### [Technical Field]

The present invention relates to a method for producing an oxidation product using manganese dioxide having a crystal structure of β-type, a catalyst for oxidation reaction containing manganese dioxide having a crystal structure of β-type, and a method for producing high surface area manganese dioxide.

### [Background Art]

Renewable biomasses such as lignocellulose and triglyceride have attracted great attention as substitutes for non-renewable fossil resources such as crude oil, coal and natural gas. From such biomasses, various high value-added products such as biofuels, commodity chemicals and bioplastics can be produced. Polyethylene furanoate (PEF) that is an analog of polyethylene terephthalate (PET) is a recyclable biomass-derived polymer having excellent characteristics such as gas barrier properties, high heat stability and low-temperature moldability. PEF is applicable to a wide range of products such as bottles, food packages and electronic materials, and its market is expected to greatly grow in near future.

The most important material for producing PEF is 2,5-furandicarboxylic acid (FDCA) that is a biomass-derived chemical substance. FDCA is obtained by oxidizing 5-hydroxymethylfurfural (HMF) in the presence of a catalyst. Regarding the catalyst used in this case, a large number of catalysts have been reported recently, and for example, cobalt acetate (Patent Literature 1), manganese acetate (Patent Literature 1), catalysts containing ruthenium, cobalt and cerium as main components (Patent Literature 2), etc. have been reported. Recently, the present inventor has reported a method for efficiently producing FDCA from HMF using activated manganese dioxide as a catalyst (Non Patent Literature 1), and manganese dioxide has been focused on as a highly active catalyst in the FDCA production.

Manganese dioxide can take a plurality of crystal structures, and crystal structures such as α-type (α-MnO₂), β-type (β-MnO₂), γ-type (γ-MnO₂), δ-type (δ-MnO₂), ε-type (ε-MnO₂), and λ-type (λ-MnO₂) are known. As a method for producing β-MnO₂ among these, a method of thermally decomposing manganese nitrate in an autoclave (Non Patent Literature 2), a method of precipitating manganese dioxide from an aqueous solution containing manganese ion that is in a subcritical state or a supercritical state (patent Literature 3), a method of immersing manganese dioxide of α-type in an acetone solution and then heating it (Patent Literature 4), or the like has been reported.

### [Citation List]

### [Patent Literature]

[Patent Literature 1]
   Japanese Patent Laid-Open No. 2011-084540
[Patent Literature 2]
   Japanese Patent Laid-Open No. 2009-001519
[Patent Literature 3]
   Japanese Patent Laid-Open No. 2007-055887
[Patent Literature 4]
   Japanese Patent Laid-Open No. 2000-211923

### [Non Patent Literature]

### [Non Patent Literature 1]

"Heterogeneously-catalyzed Aerobic Oxidation of 5-Hydroxymethylfurfural to 2,5-Furandicarboxylic Acid with Manganese Dioxide", Eri Hayashi, Tasuku Komanoya, Keigo Kamata, Michikazu Hara, ChemSusChem, 2017, 10, 654-658

### [Non Patent Literature 2]

Kaname Ito, Takehiko Takahashi, "β-nisanka mangan no kesshou no seisei to sono shinhiju (in Japanese, Production of Crystals of β-Manganese Dioxide and Its True Specific Gravity)", the Journal of Chemical Industry, Vol. 64 (1961), No. 8, pp. 1375-1378

### [Summary of Invention]

### [Technical Problem]

The method described in Non Patent Literature 1, which uses activated manganese dioxide as a catalyst, can efficiently produce FDCA, but taking into consideration an increase in demand for FDCA expected in the future, development of a method for more efficiently producing FDCA is desired. The present invention has been made under such circumstances, and it is an object of the present invention to provide manganese dioxide exhibiting higher catalytic activity.

### [Solution to Problem]

The present inventor has earnestly studied in order to solve the above problem, and as a result, the present inventor has found that manganese dioxide having a crystal structure of β-type (β-MnO₂) exhibits high catalytic activity per surface area. The specific surface area of β-MnO₂ synthesized by a conventional method is generally low, and on this account, the catalytic activity of the β-MnO₂ per mass was not so high. The present inventor has also found a method for synthesizing β-MnO₂ having a high specific surface area, and he has enabled further improvement in catalytic activity of β-MnO₂.

The present invention has been completed based on the above knowledge.

That is to say, the present invention provides the following [1] to [13].
[1] A method for producing an oxidation product by oxidizing a raw material compound in the presence of oxygen, wherein the raw material compound is oxidized in the presence of manganese dioxide having a crystal structure of β-type.
[2] The method for producing an oxidation product according to [1], wherein the oxidation of the raw material compound is carried out in a liquid phase.
[3] The method for producing an oxidation product according to [1] or [2], wherein a specific surface area of the manganese dioxide is 50 m²g⁻¹ or more.
[4] The method for producing an oxidation product according to [3], wherein the manganese dioxide is manganese dioxide produced by a method comprising the following steps:
   (1) mixing sodium permanganate and a strong acid salt of manganese(II) ion in water and stirring them;
   (2) collecting a precipitate after the step (1); and
   (3) calcining the precipitate collected in the step (2) .
[5] The method for producing an oxidation product according to [3], wherein the manganese dioxide is manganese dioxide produced by a method comprising the following steps:
   (1) mixing sodium permanganate and a weak acid salt of manganese(II) ion in water, then adding a strong acid, and thereafter stirring them;
   (2) collecting a precipitate after the step (1); and
   (3) calcining the precipitate collected in the step (2) .
[6] The method for producing an oxidation product according to any one of [1] to [5], wherein the raw material compound is an organic substance.
[7] The method for producing an oxidation product according to any one of [1] to [5], wherein the raw material compound is 5-hydroxymethylfurfural, and the oxidation product is 2,5-furandicarboxylic acid.
[8] A catalyst for oxidation reaction, comprising manganese dioxide having a crystal structure of β-type.
[9] The catalyst for oxidation reaction according to [8], wherein a specific surface area of the manganese dioxide is 50 m²g⁻¹ or more.
[10] The catalyst for oxidation reaction according to [9], wherein the manganese dioxide is manganese dioxide produced by a method comprising the following steps:
   (1) mixing sodium permanganate and a strong acid salt of manganese(II) ion in water and stirring them;
   (2) collecting a precipitate after the step (1); and
   (3) calcining the precipitate collected in the step (2) .
[11] The catalyst for oxidation reaction according to [9], wherein the manganese dioxide is manganese dioxide produced by a method comprising the following steps:
   (1) mixing sodium permanganate and a weak acid salt of manganese(II) ion in water, then adding a strong acid, and thereafter stirring them;
   (2) collecting a precipitate after the step (1); and
   (3) calcining the precipitate collected in the step (2) .
[12] A method for producing manganese dioxide, comprising the following steps:
   (1) mixing sodium permanganate and a strong acid salt of manganese(II) ion in water and stirring them;
   (2) collecting a precipitate after the step (1); and
   (3) calcining the precipitate collected in the step (2) .
[13] A method for producing manganese dioxide, comprising the following steps:
   (1) mixing sodium permanganate and a weak acid salt of manganese(II) ion in water, then adding a strong acid, and thereafter stirring them;
   (2) collecting a precipitate after the step (1); and
   (3) calcining the precipitate collected in the step (2) .

The present specification includes the contents described in the specification and/or the drawings of a patent application of Japan: Japanese Patent Application No. 2018-167523 that is the application on the basis of which the priority is claimed.

### [Advantageous Effects of Invention]

The present invention provides a novel method for producing an oxidation product using β-MnO² as a catalyst. This method enables efficient production of a useful oxidation product such as FDCA.

### [Brief Description of Drawings]

[Figure 1] Figure 1 shows scanning electron microscopic images of MnO₂ synthesized. (a) α-MnO₂, (b) β-MnO₂, (c) γ-MnO₂, (d) δ-MnO₂, (e) ε-MnO₂, (f) λ-MnO₂
[Figure 2] Figure 2 is a view showing a pathway of oxidation reaction of HMF to FDCA. In the view, rate constants of aerobic oxidation of HMF using activated MnO₂ are also shown.
[Figure 3] Figure 3 is a view showing results of analyses of MnO₂ based on H₂-TPR. (a) α-MnO₂, (b) β-MnO₂, (c) γ-MnO₂, (d) δ-MnO₂, (e) ε-MnO₂, (f) λ-MnO₂
[Figure 4] Figure 4 is a view showing a relationship between a 5-formyl-2-furancarboxylic acid (FFCA) oxidation rate (R₀) and a reduction rate estimated from H₂-TPR (■: α-MnO₂, **▲:** β-MnO₂, ●: γ-MnO₂, □: δ-MnO₂, ◆: ε-MnO₂, Δ: λ-MnO₂).
[Figure 5] Figure 5(a) shows XRD patterns of β-MnO₂-HS-1 (upper side) and β-MnO₂ (lower side, JCPDS 00-024-0735),
and Figure 5(b) shows a scanning electron microscopic image of β-MnO₂-HS-1.
[Figure 6] Figure 6 is a view showing comparison between catalytic activity of β-MnO₂ and that of β-MnO₂-HS-1 for the oxidation reaction of HMF to FDCA.

### [Description of Embodiments]

Hereinafter, the present invention will be described in detail.

### (A) Method for producing oxidation product

The method for producing an oxidation product of the present invention is a method for producing an oxidation product by oxidizing a raw material compound in the presence of oxygen, and is characterized in that the raw material compound is oxidized in the presence of manganese dioxide having a crystal structure of β-type.

The manganese dioxide is not particularly limited as long as it has a crystal structure of β-type. Whether the crystal structure is β-type or not can be judged from the X-ray diffraction pattern. Manganese dioxide having a crystal structure of β-type can be produced in accordance with a known method (e.g., hydrothermal method or sol-gel method), but it is preferably produced by a method for producing high surface area manganese dioxide described later. The reason is that the manganese dioxide produced by this method has a high specific surface area and exhibits high catalytic activity. The "high specific surface area" referred to herein usually means 50 m²g⁻¹ or more, preferably means 60 m²g⁻¹ or more, and more preferably means 70 m²g⁻¹ or more. Although the upper limit of the specific surface area does not particularly exist, but it is usually 200 m²g⁻¹ or less.

The raw material compound and the oxidation product produced are not limited to specific compounds (e.g., HMF and FDCA), and they may be any compounds as long as the compounds are a raw material compound and an oxidation product in the reaction on which manganese dioxide acts as a catalyst. The raw material compound is usually an organic substance but may be an inorganic substance. Specific examples of combinations of the raw material compound and the oxidation product produced include not only HMF and FDCA but also alcohols and aldehydes/ketones, amines and imines/nitriles, sulfides and sulfoxides, and thiols and sulfonamides, and more specific examples thereof include benzyl alcohol and benzaldehyde, 1-phenylethanol and acetophenone, benzylamine and benzonitrile, thioanisole and methyl phenyl sulfoxide, and benzenethiol and
benzenesulfonamide.

As oxygen, pure oxygen may be used, or a gas containing oxygen (e.g., air) may be used.

The method for producing an oxidation product of the present invention is usually utilized for producing a useful compound such as FDCA, but can also be utilized for, for example, removal of a harmful substance, such as a deodorization method. In this case, the raw material compound is a harmful substance such as an odorant, and the oxidation product produced is not particularly a useful compound.

In the method for producing an oxidation product of the present invention, the oxidation is usually carried out in a liquid phase, particularly in an aqueous phase, but can also be carried out in a gas phase.

In the method for producing an oxidation product of the present invention, the raw material compound is oxidized in the presence of oxygen and manganese dioxide, and in the oxidation reaction of the raw material compound, substances other than oxygen and manganese dioxide may be present. For example, when the reaction is carried out in a liquid phase, the oxidation reaction is carried out in the presence of a solvent (water, organic solvent, or mixed solvent of water and organic solvent). The oxidation reaction is usually carried out in the presence of a base. Examples of the bases include NaHCO₃, KHCO₃, Na₂CO₃. K₂CO₃, NaOH, KOH and K₃PO₄. Among these, weak bases are preferably used, and NaHCO₃ is particularly preferably used.

The amount of the manganese dioxide used is appropriately set according to the type of the raw material compound, etc. When the raw material compound is HMF, the amount of the manganese dioxide is usually 0.05 to 1.0 g, and preferably 0.1 to 0.5 g, based on 1 mmol of HMF. When the raw material compound is any of benzenethiols, the amount of the manganese dioxide is usually 0.02 to 0.5 g, and preferably 0.05 to 0.2 g, based on 1 mmol of the benzenethiol. When the raw material compound is any of benzyl alcohols, the amount thereof is usually 0.02 to 0.5 g, and preferably 0.05 to 0.2 g, based on 1 mmol of the benzyl alcohol.

The amount of the base used is appropriately set according to the types of the raw material compound and the base, etc. When the raw material compound is HMF and the base is NaHCO₃, the amount of the base is usually 0.5 to 10 mmol, and preferably 2 to 3 mmol, based on 1 mmol of HMF.

The amount of the solvent used is appropriately set according to the type of the raw material compound, etc. When the raw material compound is HMF and the solvent is water, the amount of the solvent is usually 5 to 100 mL, and preferably 10 to 50 mL, based on 1 mmol of HMF. When the raw material compound is any of benzenethiols and the solvent is a mixed solvent of DMF and water, the amount of the solvent is usually 0.2 to 5.0 mL, and preferably 0.5 to 2 mL, based on 1 mmol of the benzenethiol. When the raw material compound is any of benzyl alcohols and the solvent is toluene, the amount of the solvent is usually 0.5 to 10.0 mL, and preferably 1.0 to 5.0 mL, based on 1 mmol of the benzyl alcohol.

The partial pressure of oxygen is appropriately set according to the type of the raw material compound, etc. When the raw material compound is HMF, the partial pressure of oxygen is usually 0.1 to 10 MPa, and preferably 0.5 to 5 MPa. When the raw material compound is any of benzenethiols, the partial pressure of oxygen is usually 0.1 to 10 MPa, and preferably 0.5 to 5 MPa. When the raw material compound is any of benzyl alcohols, it is usually 0.01 to 1 MPa, and preferably 0.05 to 0.5 MPa.

The temperature in the oxidation reaction is appropriately set according to the type of the raw material compound, etc. When the raw material compound is HMF, the temperature is usually 50 to 200°C, and preferably 80 to 120°C. When the raw material compound is any of benzenethiols, the temperature is usually 50 to 200°C, and preferably 70 to 110°C. When the raw material compound is any of benzyl alcohols, the temperature is usually 10 to 100°C, and preferably 30 to 80°C.

The reaction time is appropriately set according to the type of the raw material compound, etc. When the raw material compound is HMF, the reaction time is usually 6 to 48 hours, and preferably 12 to 36 hours. When the raw material compound is any of benzenethiols, the reaction time is usually 5 to 60 hours, and preferably 10 to 50 hours. When the raw material compound is any of benzyl alcohols, the reaction time is usually 6 to 48 hours, and preferably 12 to 36 hours.

After completion of the oxidation reaction, post treatment is carried out by a known method, as needed, and a desired oxidation product can be taken out. That is to say, after completion of the oxidation reaction, post-treatment operations such as filtration, washing, extraction pH adjustment, dehydration and concentration are carried out singly or in combination of two or more, as needed, and a desired oxidation product can be taken out by concentration, crystallization, reprecipitation, column chromatography or the like.

### (B) Catalyst for oxidation reaction

The catalyst for oxidation reaction of the present invention is characterized by containing manganese dioxide having a crystal structure of β-type.

The catalyst for oxidation reaction of the present invention is usually composed of only manganese dioxide having a crystal structure of β-type, but may contain other substances.

The manganese dioxide used may be the same as the manganese dioxide used in the aforesaid method for producing an oxidation product of the present invention.

### (C) Method for producing high surface area manganese dioxide

### (C-1) First method for producing high surface area manganese dioxide

The first method for producing manganese dioxide of the present invention is characterized by comprising the following steps (1) to (3). The manganese dioxide produced by this method has a crystal structure of β-type and has a high specific surface area. The manganese dioxide produced by this first method for producing manganese dioxide is sometimes referred to as "β-MnO₂-HS-1".

In the step (1), sodium permanganate and a strong acid salt of manganese(II) ion are mixed in water and stirred.

Examples of the strong acid salts of manganese(II) ion include manganese sulfate and manganese nitrate.

The amount of water used is not particularly limited, but it is usually 1 to 40 mL, and preferably 5 to 20 mL, based on 1 mmol of sodium permanganate.

The stirring time is not particularly limited either, but it is usually 1 to 40 min, and preferably 10 to 30 min.

Through this step, amorphous manganese dioxide is produced.

In the step (2), a precipitate is collected after the step (1).

The collection can be carried out by filtration or the like. The collected precipitate is usually washed with water or the like, subsequently dried and thereafter used in the next step.

In the step (3), the precipitate collected in the step (2) is calcined.

The calcining temperature is not particularly limited, but it can be set to, for example, 200 to 600°C.

The calcining time is not particularly limited either, but it can be set to, for example, 2 to 10 hours.

### (C-2) Second method for producing high surface area manganese dioxide

The second method for producing manganese dioxide of the present invention is characterized by comprising the following steps (1) to (3). The manganese dioxide produced by this method also has a crystal structure of β-type and has a high specific surface area. The manganese dioxide produced by this second method for producing manganese dioxide is sometimes referred to as "β-MnO₂-HS-2".

In the step (1), sodium permanganate and a weak acid salt of manganese(II) ion are mixed in water, then a strong acid is added, and thereafter, they are stirred.

The weak acid salt of manganese(II) ion is, for example, manganese acetate. Examples of the strong acids include sulfuric acid and nitric acid.

The amount of water used is not particularly limited, but it is usually 1 to 40 mL, and preferably 5 to 20 mL, based on 1 mmol of sodium permanganate.

The amount of the strong acid added is not particularly limited, but the strong acid is usually added in such an amount that pH becomes 1.5 or less, and is preferably added in such an amount that pH becomes 0.8 or less.

The stirring time is not particularly limited either, but it is usually 1 to 120 min, and preferably 10 to 60 min.

Through this step, amorphous manganese dioxide is produced.

In the step (2), a precipitate is collected after the step (1).

The collection can be carried out by filtration or the like. The collected precipitate is usually washed with water or the like, subsequently dried and thereafter used in the next step.

In the step (3), the precipitate collected in the step (2) is calcined.

The calcining temperature is not particularly limited, but it can be set to, for example, 200 to 600°C.

The calcining time is not particularly limited either, but it can be set to, for example, 2 to 10 hours.

### [Examples]

Hereinafter, the present invention will be described in more detail with reference to examples, but the present invention is in no way limited to these examples.

### [Example 1]

### (A) Experimental method

### (1) Experimental instrument

On a diffractometer (Ultima IV, Rigaku; Cu K α, λ=1.5405 Å, 40 kV-40 mA) equipped with a high-speed one-dimensional detector (D/teX Ultra, Rigaku), an XRD pattern was recorded. The diffraction data were collected in the range of 2θ = 10 to 80° at a scanning rate of 20° min⁻¹ and a step of 0.02°. ICP-AES analysis was carried out by Shimadzu ICPS-8100 spectrometer. A nitrogen adsorption-desorption isotherm was measured at 77 K using a surface area analyzer (Nova-4200e, Quantachrome). Before the measurement, the sample was heated at 423 K for 1 hour under vacuum to remove physisorbed water. Brunauer-Emmett-Teller (BET) surface area was estimated over a relative pressure (P/P0) range of 0.05 to 0.30. The shape of the sample was examined using a scanning electron microscope (SEM; S-5500, Hitachi). TG-DTA measurement was carried out using a differential thermal analyzer (TG8120, Rigaku). TG-DTA analysis was carried out at a heating rate of 10 K min⁻¹ from room temperature to 1273 K in a stream of N₂ (200 mL min⁻¹). XPS analysis was carried out using Shimadzu ESCA-3400HSE spectrometer using Mg Kα radiation (1253.6 eV) at 10 kV and 25 mA. The sample was pressed to prepare pellets, and the pellets were fixed onto a double-sided carbon tape. The binding energy was calibrated using C 1s band of 284.6 eV. Using the XPS Peak 4.1 program, the spectrum was fitted and evaluated, but the background was subtracted using a Shirley function. The deconvoluted Mn 2p spectrum of MnO₂ shows three peaks having binding energies of 640.8, 641.8, 642.8 and 644.5 eV corresponding to Mn^{II}, Mn^{III}, Mn^{IV} and shake-up peak, respectively. Using Mettler Toledo Easy Pro Titrator System, iodine titration was carried out. About 10 mg of MnO₂ was added to a mixture of a 0.5 M HCl aqueous solution (12 mL) and a 2 M KI aqueous solution (5 mL), and the resulting solution was titrated with a 0.01 M Na₂S₃O₃ aqueous solution. H₂TPR profile was measured by BEL Japan BELCAT-A chemisorption analyzer equipped with a thermal conductivity detector (TCD). In a quartz cell, 50 mg of a sample was placed, and subsequently, the sample was heated at a rate of 10 K min⁻¹ from 323 K to 923 K in a stream of 5% H₂/Ar (50 mL min⁻¹). The initial reduction rate was estimated in a range of 553 K up to 593 K, this corresponded to a range of less than 10% of a lattice oxygen atom having been reduced by H₂, and all the MnO₂ catalysts were stable in this range. Using a photo diode array (PDA) and a refractive index (RI) detector, high performance liquid chromatography (HPLC; LC-2000, Jasco) analysis was carried out with Aminex HPX-87H column (7.8 mm diameter × 300 mm, Bio-Rad Laboratories, Inc. Co. Ltd.; eluent (0.5 mM H₂SO₄), flow rate (0.5 mL/min), column temperature (308 K)). The retention times of FDCA, 5-hydroxymethyl-2-furancarboxylic acid (HMFCA), FFCA, HMF and 2,5-diformyl furan (DFF) were 20.9, 27.3, 29.4, 41.7, and 52.1 minutes, respectively. The crystal structure was drawn by utilizing visualization of the electronic and structural analysis (VESTA) program.

### (2) Experimental method for oxidation reaction

Oxidation reaction was carried out using a 30 mL capped test tube or an autoclave reaction vessel having a 13 mL Teflon (registered trademark) container. Typical oxidation reaction of HMF was carried out in accordance with the following procedure. Into the autoclave reaction vessel, HMF (0.2 mmol), MnO₂ (0.05 g), NaHCO₃ (0.6 mmol), pure water (5 mL) and O₂ (1 MPa) were introduced, and they were allowed to react at 100°C for 24 hours. After the reaction, the catalyst was separated by filtration, and the filtrate was diluted to 10 times with pure water and analyzed by HPLC. The collected catalyst was washed with pure water (25 mL), then dried at 80°C, and reused for the experiment.

### (3) Synthesis of high surface area β-MnO₂ (β-MnO₂-HS-1)

β-MnO₂-HS-1 was synthesized in accordance with the following procedure. To an aqueous solution (20 mL) of NaMnO₄ (1.28 g, 4 mmol) having been vigorously stirred, an aqueous solution (20 mL) of MnSO₄·5H₂O (1.45 g, 6 mmol) was dropwise added, and after addition of all the solution, they were stirred for 10 minutes. A precipitate was collected by filtration, washed with pure water (300 mL), and then dried at 353 K overnight. The dried precipitate was calcined at 400°C for 5 hours, whereby β-MnO₂-HS-1 that was a black powder was obtained. Yield: 0.83 g (96%)

A surface area of the resulting β-MnO₂-HS-1 was 73 m²g⁻¹, and an average pore diameter thereof was 3.5 nm.

### (4) Synthesis of β-MnO₂ by hydrothermal method

β-MnO₂ was synthesized in accordance with the procedure previously reported (Y.Y. Gorbanev, S. Kegnaes, A. Riisager, Top. Catal. 2011, 54, 1318-1324). In water (40 mL), NaMnO₄ (1.28 g, 4 mmol) was dissolved, to the solution was added MnSO₄·5H₂O (2.89 g, 12 mmol) while stirring, and they were further stirred for 30 minutes. This mixture was transferred into a stainless steel autoclave equipped with a Teflon (registered trademark) inner cylinder type closed container (TAF-SR type, Taiatsu Techno Corporation). The mixture was heated at 433 K for 12 hours, and thereafter, a precipitate was collected, washed with water (300 mL) and dried at 353 K overnight, thereby obtaining a black powder of β-MnO₂. Yield: 1.66 g (83%)

### (B) Experimental result

### (1) Effect of MnO₂ crystal structure on oxidation reaction of HMF to FDCA

Syntheses and characterizations of MnO₂ having various crystal structures were carried out, and their oxidation catalytic action was studied. α-, β-, γ-, δ-, ε- and λ-MnO₂ were synthesized. From XRD analysis of each sample, production of tetragonal crystal α-MnO₂, tetragonal crystal β-MnO₂, orthorhombic crystal γ-MnO₂, trigonal crystal δ-MnO₂, hexagonal crystal ε-MnO₂, and cubic crystal λ-MnO₂ was confirmed. Metal content, water content, and average oxidation state (AOS) of the Mn species were determined by ICP-AES, TG-DTA, and iodine reduction titration, respectively. The results are set forth in Table 1. The amount of Mn in every MnO₂ catalyst was 47.86 to 62.39 wt%. The AOS of each of the β-, γ- and ε-MnO₂ was about 4, while the AOS of each of the α-, δ- and λ-MnO₂ was smaller (3.70 to 3.81) because of influence of charge compensation due to the presence of K⁺ ion.

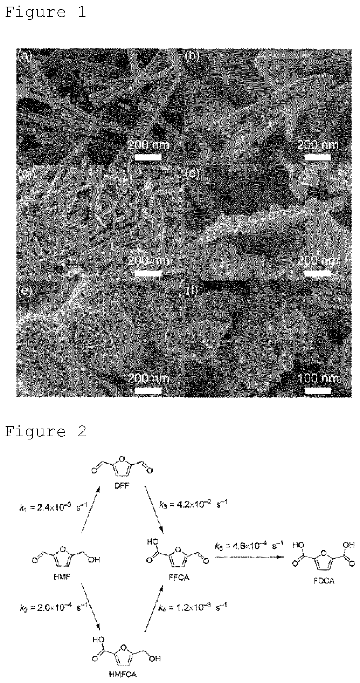

Scanning electron microscopic (SEM) images of the synthesized MnO₂ are shown in Figure 1. The shapes and the average sizes are listed in Table 1. The α-, β- and γ-MnO₂ were each composed of rod-like particles, and the particle diameter depended upon the sample. The δ- and λ-MnO₂ were each composed of spherical particles, and the ε-MnO₂ was composed of rose-like particles. Regarding the specific surface areas of MnO₂ calculated from the BET plot of the N₂ adsorption isotherm (77 K), the specific surface area of the ε-MnO₂ (181 m²g-¹) was considerably large, but the specific surface areas of the α-, β-, γ-, δ- and λ-MnO₂ were of medium values to low values (14 to 67 m²g⁻¹). These results were in good agreement with the average particle diameters obtained from the SEM observation.

**[Table 1]**

| Bulk content, surface area. particle shape and particle size of MnO₂ catalyst ^{a} | | | |
|---|---|---|---|
| Catalyst Bulk content (wt%) | Specific surface area (m²g⁻¹) | Particle shape | Particle size (nm) |
| | | | 30-60 wide |
| *α*-MnO₂ Mn: 56.75: K: 7.12: water: 0.82 | 30 | rod-like ^{a} | 60-1000 long |
| | | | 50-70 wide |
| *β*-MnO₂ Mn: 60.81; water; 0,46 | 14 | rod-like^{a} | 150-1000 long |
| | | | 10-60 wide |
| γ-MnO₂ Mn: 62.03: water: 2.45 | 39 | rod-iike ^{a} | 40-800 long |
| δ-MnO₂ Mn: 52.43: K: 8.67; water: 6.86 | 35 | spherical ^{b} | 50-150 |
| | | rose-like ^{c} | 10-20 thick |
| *ε* -MnO₂ Mn: 47.86; Fe: 6.1: water: 4.84 | 181 | (constituted of nanoplates) | 60-200 wide |
| *λ*-MnO₂ Mn: 62.39: water: 3.76 | 67 | spherical^{b} | 40-70 |

| | | | |
|---|---|---|---|
| ^{a} The nano rod particle size means a diameter and a length. ^{b} average particle diameter, ^{c} thickness and length of nanoplate. ^{d} particle size based on SEM observation | | | |

Using the MnO₂ catalysts having various crystal structures, oxidation reaction of HMF using O₂ (1 MPa) as an oxidizing agent in the presence of NaHCO₃ (3 equiv. based on HMF) was studied. The results are set forth in Table 2. In every case, the HMF conversion ratio was in the range of 93 to 99%. Among the catalysts studied, the activated MnO₂, the α-MnO₂ and the ε-MnO₂ exhibited FDCA yields of 59 to 74%. On the other hand, the β-, γ-, δ-and λ-MnO₂ exhibited high HMF conversion ratios, but as a main product, FFCA was produced in a ratio of 60 to 69%, and the FDCA yields were of low values (5 to 28%). In order to confirm stability of the MnO₂ catalysts, XRD pattern of each catalyst after the reaction and the amount of a metal in the filtrate based on the ICP-AES analysis were measured. In all the catalysts except the ε-MnO₂, elution of Mn species into the filtrate was not confirmed. There was almost no change in the positions of the XRD peaks of the α-, β- and ε-MnO₂ between before and after the reaction, but the peak intensities of the α- and β-MnO₂ slightly increased. In the γ-MnO₂, peak shift accompanying the unit cell extension due to H insertion was confirmed. On the other hand, the XRD patterns of the δ- and λ-MnO₂ greatly changed after the reaction. In the δ-MnO₂, the intensities of (003) and (006) peaks assigned to layer structure greatly decreased, and elution of K⁺ ion of 55% into the filtrate was confirmed. Moreover, in the XRD pattern of the λ-MnO₂ after the reaction, peaks having been observed before the reaction almost disappeared. These results have suggested that the structures of the δ- and λ-MnO₂ are unstable under the present reaction conditions.

**[Table 2]**

| | | | | | |
|---|---|---|---|---|---|
| Influence of MnO₂ catalyst on oxidation of HMF to FDCA^{a} | | | | | |
| 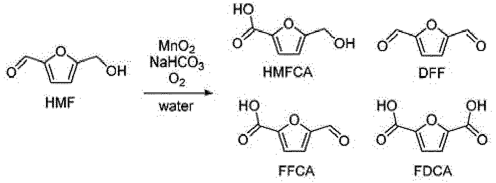 | | | | | |

| | | Yield (%) | | | |
|---|---|---|---|---|---|
| Catalyst | Conversion ratio (%) | HMFCA | DFF | FFCA | FDCA |
| Activated MnO₂ | >99 | - | - | 15 | 74 |
| *α*-MnO₂ | >99 | 1 | 0 | 36 | 59 |
| *β*-MnO₂ | >99 | 1 | - | 66 | 28 |
| *γ*-MnO₂ | 93 | 2 | 3 | 60 | 5 |
| *δ*-MnO₂ | 96 | - | 1 | 69 | 10 |
| *ε*-MnO₂ | >99 | 1 | - | 27 | 63 |
| *λ*-MnO₂ | >99 | - | - | 66 | 12 |

| | | | | | |
|---|---|---|---|---|---|
| ^{a} Reaction conditions: catalyst (0.05 g), HMF (0.2 mmol). NaHCO₃ (0.6 mmol), water (5 mL), *p*O₂ (1 MPa), 373 K, 24 h. | | | | | |

### (2) Reaction mechanism in oxidation reaction of HMF to FDCA with activated MnO₂ catalyst

Since the oxidation of HMF to FDCA is a complicated successive reaction, it is difficult to evaluate fundamental reactivity of the MnO₂ catalyst. Then, determination of a key step in the HMF oxidation with the activated MnO₂ catalyst was carried out by the kinetic analysis. Figure 2 shows a reaction pathway from HMF to FDCA. By the oxidation of a hydroxyl group and a formyl group of HMF, DFF and HMFCA are given, respectively. By the successive oxidation of DFF and HMFCA, FFCA is obtained, and FFCA is finally oxidized into FDCA. The reaction pathway from HMF to FFCA depends upon the catalytic system, and the mechanism via HMFCA has been reported in an Au, Pt, Ru, or Pd supported catalyst/NaOH/O₂ reaction system, while the mechanism via DFF and HMFCA has been reported in a Pt/C catalyst/NaHCO₃/O₂ reaction system.

Assuming that every step is a reaction of primary dependence on a substrate, rate constants (k₁ to k₅ in Figure 2) are estimated from fitting to time changes, and in the activated MnO₂ catalyst, the estimation resulted in k₁ = 2.4×10⁻³, k₂ = 2.0×10⁻⁴, k₃ = 4.2×10⁻², k₄ = 1.2×10⁻³, and k₅ = 4.6×10⁻⁴. k₂ is about 10 times smaller than K₁, and this suggests that the mechanism from HMF to FFCA is mechanism via DFF. This is also supported by oxidation of alcohol with MnO₂ being generally faster than aldehyde oxidation. From the fact that the value of k₅ is smaller than the values of k₁, k₃ and k₄, it has been suggested that the oxidation reaction of FFCA to FDCA is a rate-determining step in the present reaction.

### (3) Influence of MnO₂ crystal structure on oxidation of FFCA to FDCA

By comparing rates (Ro) of oxidation reaction of FFCA to FDCA, the reaction being a rate-determining step of the oxidation reaction of HMF to FDCA, on the basis of the results of the kinetic analysis, a structure-reactivity correlation in the oxidation reaction with a MnO₂ catalyst was studied (Table 3). The initial rate per surface area decreased in the order of β-MnO₂ (16.4 µmol h⁻¹ m⁻²) > λ-MnO₂ (12.2 µmol h⁻¹ m⁻²) > α-MnO₂ (7.6 µmol h⁻¹ m⁻²) > γ-MnO₂ (7.4 µmol h⁻¹ m⁻²) > δ-MnO₂ (5.3 µmol h⁻¹ m⁻²) > ε-MnO₂ (2.3 µmol h⁻¹ m⁻²).

**[Table 3]**

| | | | |
|---|---|---|---|
| Influence of synthetic MnO₂ catalyst on oxidation of FFCA to FDCA ^{a} | | | |
| 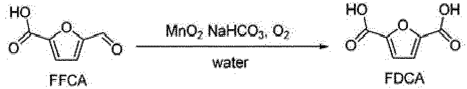 | | | |

| FDCA | | | |
|---|---|---|---|
| Catalyst | Conversion ratio (%) | Yield (%) | *R*₀ (*µ* mol h⁻¹ m⁻²) |
| Activated MnO₂ | 30 | 34 | 5.6 |
| *α*-MnO₂ | 8 | 12 | 7.6 |
| *β*-MnO₂ | 7 | 12 | 16.4 |
| *γ*-MnO₂ | 10 | 14 | 7.4 |
| *δ*-MnO₂ | 6 | 9 | 5.3 |
| *ε*-MnO₂ | 16 | 21 | 2.3 |
| *λ*-MnO₂ | 47 | 50 | 12.2 |

| | | | |
|---|---|---|---|
| ^{a} Reaction conditions: catalyst (0.05 g), FFCA (0.2 mmol), NaHCO₃ (0.6 mmol), water (5 mL), *p*O₂ (1 MPa). 373 K. 2 h. | | | |

In order to confirm excellent FFCA oxidation capacity of the β-MnO₂ among various MnO₂, XPS measurement was carried out. Peak separation results of spectra of the MnO₂ catalysts are set forth in Table 4. As surface species, Mn⁴⁺, Mn³⁺ and Mn²⁺ species were observed, and the surface Mn valences were estimated to be 3.00 to 3.57. The O 1s peak was separable into three peaks assigned to lattice oxygen, adsorbed oxygen and adsorbed water. It has been reported that the valence of a metal or the state of adsorbed oxygen species plays an important role in the oxidation reaction with a metal oxide catalyst, but a correlation between the reactivity and the surface Mn valence or the amount of adsorbed oxygen has not been confirmed. From these results, the reactivity cannot be explained only by the above two factors, and it is thought that a difference in fundamental reactivity of an oxygen atom accompanying the crystal structure plays an important role in the present oxidation reaction.

**[Table 4]**

| | | | | | |
|---|---|---|---|---|---|
| Binding energy fraction, and average oxidation state of synthetic MnO₂ catalyst^{a} | | | | | |

| | Binding energy of Mn 2p (eV) | | Binding energy of O 1s (eV) | | |
|---|---|---|---|---|---|
| Catalyst | Mn (IV) | Mn (III) | Lattice oxygen | Adsorbed oxygen | Adsorbed water |
| *α*-MnO₂ | 642.36 (54%) | 641.36 (46%) | 529.5 (77%) | 531.2 (19%) | 533.0 (4%) |
| *β*-MnO₂ | 642.23 (44%) | 641.23 (56%) | 529.2 (74%) | 5312 (23%) | 533.0 (3%) |
| *γ*-MnO₂ | 642.15 (54%) | 641.15 (46%) | 529.4 (71%) | 531.2 (25%) | 533.0 (4%) |
| *δ*-MnO₂ | 642.46 (36%) | 641,46 (64%) | 529.7 (73%) | 531.4 (21%) | 533.0 (6%) |
| *ε*-MnO₂*^{b}* | 642.44 (32%) | 641.44 (38%) | 529.5 (69%) | 531.4 (27%) | 533.0 (4%) |
| *λ*-MnO₂ | 642.17 (53%) | 641.17 (47%) | 529.6 (71%) | 531.4 (25%) | 533.0 (4%) |

| | | | | | |
|---|---|---|---|---|---|
| ^{a} Each value in parentheses is a percentage of a peak, ^{b} Mn²⁺ species was observed on a surface of ε-MnO₂. | | | | | |

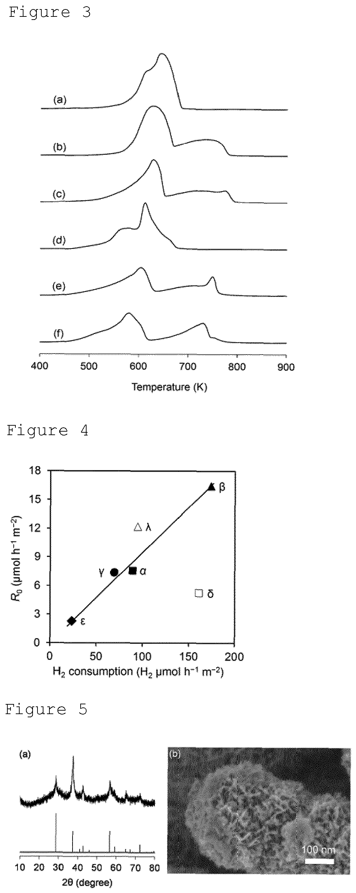

The results of analyses of MnO₂ based on the H₂ temperature-programmed reduction method (H₂-TPR) are shown in Figure 3. Reduction of all the MnO₂ catalysts started from temperatures of 410 to 470 K, and two main reduction peaks assigned to (i) reduction of MnO₂ to Mn₃O₄ (via Mn₂O₃) and (ii) reduction of Mn₃O₄ to MnO were confirmed. The peak top temperature was different for each crystal phase, and a good correlation between the peak top temperature and the reactivity was not confirmed. It has been reported that owing to an increase in diffusion resistance due to a decrease in catalyst surface area, the reduction peak top temperature shifts to a higher temperature, and that the oxidation reaction rate is correlated with the initial reduction rate. Actually, the reactivity depended upon not the reduction peak top temperature but the initial reduction rate, and the reduction rate per surface area estimated from the H₂-TPR decreased in the order of β-MnO₂ (174 H₂ µmol h⁻¹ m⁻²) > δ-MnO₂ (161 H₂ µmol h⁻¹ m⁻²) > λ-MnO₂ (95 H₂ µmol h⁻¹ m⁻²) > α-MnO₂ (89 H₂ µmol h⁻¹ m⁻²) > γ-MnO₂ (69 H₂ µmol h⁻¹ m⁻²) > ε-MnO₂ (23 H₂ µmol h⁻¹ m⁻²). A plot of the reduction rate against the reaction rate of the FFCA oxidation is shown in Figure 4. Except for unstable δ- and λ-MnO₂, an almost linear relationship was confirmed, and it is thought that the oxidation of a substrate supports mechanism of reaction that is promoted by an oxygen atom in MnO₂.

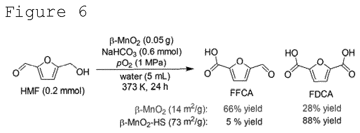

Synthesis of β-MnO₂ by hydrothermal method or sol-gel method has been reported, but their specific surface areas are generally low and restrict catalytic performance. The present inventor has developed a novel synthesis method for β-MnO₂ and succeeded in improvement of a specific surface area up to 73 m²g⁻¹ from 14 m²g⁻¹. By calcining an amorphous precursor obtained by mixing Na[MnO₄] and Mn(SO₄) in a molar ratio of 2:3, pure high surface area β-MnO₂ (β-MnO₂-HS-1) was obtained. The XRD pattern for the β-MnO₂-HS-1 was in good agreement with that of tetragonal crystal β-MnO₂ (Figure 5(a)), and the SEM image showed a rose-like particle constituted of small nanoplates (Figure 5(b)). When the β-MnO₂-HS-1 was applied to FDCA synthesis due to HMF oxidation, the FDCA yield for 24 hours was 88% and was greatly improved as compared with that of β-MnO₂ synthesized by hydrothermal method (Figure 6).

### [Example 2]

### (1) Sulfonamide synthesis reaction

Oxidation reaction was carried out using an autoclave reaction vessel having a 13 mL Teflon (registered trademark) container. Typical sulfonamide synthesis reaction was carried out in accordance with the following procedure. Into the autoclave reaction vessel, benzenethiol (1.0 mmol), MnO₂ (0.1 g), a 28% NH₃ aqueous solution (5.0 mmol), DMF/H₂O (0.6/0.4 mL), and O₂ (1 MPa) were introduced, and they were allowed to react at 90°C for 20 hours. After the reaction, the catalyst was separated by filtration, and isolation and collection of a product in the filtrate were carried out using an elution position control automated setup medium pressure preparative liquid chromatograph (EPCLC-AI-580S, manufactured by Yamazen Corporation).

The yields of benzenesulfonamides produced are set forth in the following table.

**[Table 5]**

| Oxidative sulfonamidation of compound 1 to compound 2 using β-MnO₂-HS-1 ^{[a]} | | | |
|---|---|---|---|
| entry | substrate | product | yield^{[b]} (%) |
| 1 | 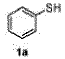 | 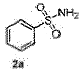 | 88% |
| 2 | 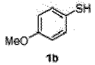 | 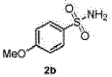 | 88% |
| 3 | 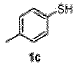 | 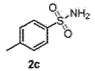 | 82% |
| 4^{[*c*]} | 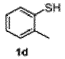 | 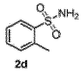 | 83% |
| 5 | 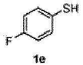 | 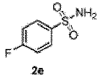 | 80% |
| 6 | 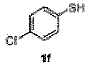 | 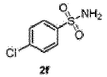 | 85% |
| 7 | 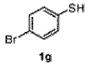 | 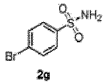 | 82% |
| 8^{[d]} | 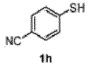 | 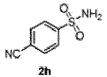 | 68% |
| 9^{[c]} | 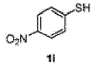 | 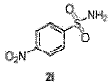 | 69% |
| 10^{[c]} | 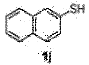 | 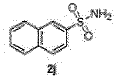 | 41% |

| | | | |
|---|---|---|---|
| [a] Reaction conditions: β-MnO₂-HS-1 (0.1 g), thiols (1 mmol), DMF/water (0.6/0.4 mL)_{.} 28% NH₃ aqueous solution (5 mmol), ρO₂ (1 MPa), 90°C, 20 hours [b] Isolated yield; In every reaction, the conversion ratio of the compound 1 became 99% or more, Yield (%) = product (mol)/initial substrate (mol) × 100 [c] The reaction time was set to 40 hours. [d] DMF (1 mL) was used. Instead of the NH₃ aqueous solution, NH₃ gas (0.5 MPa) was used. [e] β-MnO₂-HS-1 (0.15 g) was used. | | | |

### (2) Oxidation reaction of alcohol

Oxidation reaction was carried out using a 30 mL capped test tube. Typical alcohol oxidation reaction was carried out in accordance with the following procedure. Into the test tube, benzyl alcohol (1.0 mmol), MnO₂ (0.1 g), toluene (2 mL) and O₂ (0.1 MPa) were introduced, and they were allowed to react at 50°C for 24 hours. Qualification and quantification of the reaction products were carried out using a gas chromatograph.

**[Table 6]**

| Oxidation of alcohol to aldehyde using β-MnO₂-HS-1 ^{[a]} | | | |
|---|---|---|---|
| entry | substrate | product | yield (%) |
| 1 | 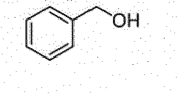 | 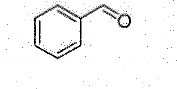 | 98% |
| 2 | 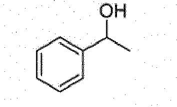 | 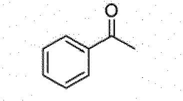 | 94% |
| 3 | 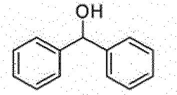 | 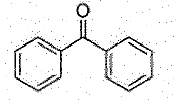 | >99% |

| | | | |
|---|---|---|---|
| [a] Reaction conditions: β-MnO₂-HS-1 (0.1 g), alcohols (1 mmol), toluene (2 mL), ρO₂ (0.1 MPa), 50°C, 24 hours; In every reaction, the conversion ratio of each alcohol became 99% or more. Yield (%) = product (mol)/initial substrate (mol) × 100 | | | |

### (3) Synthesis of high surface area β-MnO₂ (β-MnO₂ -HS-2)

β-MnO₂-HS-2 was synthesized in accordance with the following procedure. To an aqueous solution (20 mL) of NaMnO₄·H₂O (0.64 g, 4 mmol) having been vigorously stirred, an aqueous solution (20 mL) of Mn(OAc)₂·4H₂O (1.47 g, 6 mmol) was dropwise added, and a 0.5 M H₂SO₄ aqueous solution was added to adjust pH to 0.74, following by stirring for 30 minutes. A precipitate was collected by filtration, washed with pure water (2 L), and then dried at 80°C overnight. The dried precipitate was calcined at 400°C for 5 hours, whereby β-MnO₂-HS-2 that was a black powder was obtained. Yield: 0.8852 g (82%)

A surface area of the resulting β-MnO₂-HS-2 was 129 m²g⁻¹, and an average pore diameter thereof was 5 to 7 nm.

All the publications, the patents and the patent applications cited in the present specification are incorporated into the present specification as references, as they are.

### [Industrial Applicability]

Since the method for producing an oxidation product of the present invention can be used for producing useful substances such as FDCA, this method is applicable in industrial fields related to production of such substances.

## Claims

1. A method for producing an oxidation product by oxidizing a raw material compound in the presence of oxygen, wherein the raw material compound is oxidized in the presence of manganese dioxide having a crystal structure of β-type.

2. The method for producing an oxidation product according to claim 1, wherein the oxidation of the raw material compound is carried out in a liquid phase.

3. The method for producing an oxidation product according to claim 1 or 2, wherein a specific surface area of the manganese dioxide is 50 m²g⁻¹ or more.

4. The method for producing an oxidation product according to claim 3, wherein the manganese dioxide is manganese dioxide produced by a method comprising the following steps:
(1) mixing sodium permanganate and a strong acid salt of manganese(II) ion in water and stirring them;
(2) collecting a precipitate after the step (1); and
(3) calcining the precipitate collected in the step (2) .

5. The method for producing an oxidation product according to claim 3, wherein the manganese dioxide is manganese dioxide produced by a method comprising the following steps:
(1) mixing sodium permanganate and a weak acid salt of manganese(II) ion in water, then adding a strong acid, and thereafter stirring them;
(2) collecting a precipitate after the step (1); and
(3) calcining the precipitate collected in the step (2) .

6. The method for producing an oxidation product according to any one of claims 1 to 5, wherein the raw material compound is an organic substance.

7. The method for producing an oxidation product according to any one of claims 1 to 5, wherein the raw material compound is 5-hydroxymethylfurfural, and the oxidation product is 2,5-furandicarboxylic acid.

8. A catalyst for oxidation reaction, comprising manganese dioxide having a crystal structure of β-type.

9. The catalyst for oxidation reaction according to claim 8, wherein a specific surface area of the manganese dioxide is 50 m²g⁻¹ or more.

10. The catalyst for oxidation reaction according to claim 9, wherein the manganese dioxide is manganese dioxide produced by a method comprising the following steps:
(1) mixing sodium permanganate and a strong acid salt of manganese(II) ion in water and stirring them;
(2) collecting a precipitate after the step (1); and
(3) calcining the precipitate collected in the step (2) .

11. The catalyst for oxidation reaction according to claim 9, wherein the manganese dioxide is manganese dioxide produced by a method comprising the following steps:
(1) mixing sodium permanganate and a weak acid salt of manganese(II) ion in water, then adding a strong acid, and thereafter stirring them;
(2) collecting a precipitate after the step (1); and
(3) calcining the precipitate collected in the step (2) .

12. A method for producing manganese dioxide, comprising the following steps:
(1) mixing sodium permanganate and a strong acid salt of manganese(II) ion in water and stirring them;
(2) collecting a precipitate after the step (1); and
(3) calcining the precipitate collected in the step (2) .

13. A method for producing manganese dioxide, comprising the following steps:
(1) mixing sodium permanganate and a weak acid salt of manganese(II) ion in water, then adding a strong acid, and thereafter stirring them;
(2) collecting a precipitate after the step (1); and
(3) calcining the precipitate collected in the step (2) .
